(19) Europäisches Patentamt
European Patent Office
Office européen des brevets

(11) Veröffentlichungsnummer: **0 430 055 A1**

# (12) EUROPÄISCHE PATENTANMELDUNG

(21) Anmeldenummer: **90122258.8**

(51) Int. Cl.5: **C07C 43/305**, C25B 3/02

(22) Anmeldetag: **22.11.90**

(30) Priorität: **28.11.89 DE 3939285**

(43) Veröffentlichungstag der Anmeldung:
**05.06.91 Patentblatt 91/23**

(84) Benannte Vertragsstaaten:
**BE DE ES FR GB IT NL Patentblatt**

(71) Anmelder: **BASF Aktiengesellschaft**
**Carl-Bosch-Strasse 38**
**W-6700 Ludwigshafen(DE)**

(72) Erfinder: **Hermeling, Dieter, Dr.**
**Zum Ordenswald 73 f**
**W-6730 Neustadt(DE)**
Erfinder: **Hickmann, Eckhard, Dr.**
**Kantstrasse 23**
**W-6701 Dannstadt-Schauernheim(DE)**
Erfinder: **Koehler, Ulrich, Dr.**
**Werderstrasse 48**
**W-6900 Heidelberg(DE)**

## (54) 2-tert.-Butyl-p-benzochinontetraalkylketale und ihre Herstellung.

(57) 2-tert.-Butyl-p-benzochinontetraalkylketale der Formel

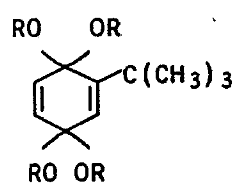

I,

in der R einen Methyl- oder Ethylrest bedeutet, und ihre Herstellung durch Elektrooxidation von 2-tert.-Butylphenylalkylether der Formel

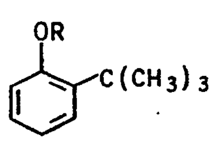

II.

EP 0 430 055 A1

## 2-TERT.-BUTYL-P` BENZOCHINONTETRAALKYLKETALE UND IHRE HERSTELLUNG

Diese Erfindung betrifft neue 2-tert.-Butyl-p-benzochinontetraalkylketale und ein verfahren zu deren Herstellung. Die neuen 2-tert.-Butyl-p-benzochinontetraalkylketale haben die Formel

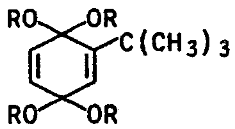

I,

in der R einen Methyl- oder Ethylrest bedeutet. Die neuen 2-tert.-Butyl-p-benzochinontetraalkylketale sind Vorprodukte des 2-tert.-Butylhydrochinons, das als Antioxidans in der Lebensmittelindustrie Verwendung findet.

Die Herstellung von 2-tert.-Butylhydrochinon durch Umsetzung von Hydrochinon mit Isobutylen ist z.B. in den US-Patentschriften 2 722 556 und 4 323 714 beschrieben.

Aus Coll. Czech. Chem. Commun. 29, 381-389 (1964), geht hervor, daß bei dieser Reaktion u.a. Nebenprodukte durch Oxidation des Hydrochinons und durch Umwandlung des Alkylierungsmittels z.B. in Diisobutylen entstehen. In J. Org. Chem. 49, 4161-4165 (1984), wird beschrieben, daß 2-tert.-Butylhydrochinon erhalten wird, wenn das Alkylierungsmittel nicht im Überschuß eingesetzt wird. Andernfalls entsteht 2,5-Di-tert.-butylhydrochinon. Diese Nebenproduktsituation wird auch in DE 11 59 961 hervorgehoben.

Besonders gravierend ist das Problem durch den Umstand, daß das 2-tert.-Butylhydrochinon in der Lebensmittelindustrie verwendet wird, so daß an das Produkt besondere Reinheitsanforderungen gestellt werden müssen.

Die Notwendigkeit besonderer Maßnahmen in dieser Richtung wird u.a. dadurch deutlich, daß in der jap. Offenlegungsschrift 81 338/87 ein Verfahren zur Herstellung und in der jap. Offenlegungsschrift 81 339/87 sowie in der jap. Offenlegungsschrift 81 340/87 ein Verfahren zur Reinigung von 2-tert.-Butylhydrochinon beschrieben wird.

Es wurde nun gefunden, daß man 2-tert.-Butylhydrochinon in einfacher Weise durch Verseifung und Reduktion von 2-tert.-Butyl-p-benzochinontetraalkylketalen herstellen kann, da die Ketale frei von isomeren Nebenprodukten in hohen Reinheiten isoliert werden können, so daß letztlich ein sehr reines 2-tert.-Butylhydrochinon erhalten wird.

Die 2-tert.-Butyl-p-benzochinontetraalkylketale der Formel I hinwiederum lassen sich besonders vorteilhaft dadurch herstellen, daß man 2-tert.-Butylphenylalkylether der Formel

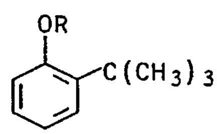

II,

in der R für einen Methyl- oder Ethylrest steht, in Gegenwart eines Alkohols ROH, in der R die genannte Bedeutung hat, elektrochemisch oxidiert.

Aus der DE-OS 36 19 656 ist bekannt, daß 2,6-Dimethylphenylalkylether anodisch in guten Selektivitäten zu 2,6-Dimethyl-p-benzochinontetraalkylketalen oxidiert werden können. Die hohen Selektivitäten lassen sich durch die Blockierung der beiden zur Alkoxigruppe ortho-ständigen Methylgruppen begründen; denn aus Tetrahedron 29, 279-285 (1973), geht hervor, daß die anodische Methoxilierung von Anisol zu einem Isomerengemisch aus o-, m- und p-Dimethoxybenzol im Verhältnis 39:<3:58 führt. Durch Weiteroxidation von o- bzw. p-Dimethoxybenzol erhält man schließlich die entsprechenden o- bzw. p-Benzochinontetramethylketale.

Aufgrund dieser Kenntnisse war es sehr überraschend, daß die anodische Alkoxilierung von 2-tert.-Butylphenylalkylethern ohne Bildung der zu erwartenden 3-tert.-Butyl-o-benzochinontetraalkylketal-Nebenprodukte selektiv zum 2-tert.-Butyl-p-benzochinontetraalkylketalen führte.

Die erfindungsgemäße Elektrooxidation kann in an sich üblichen Elektrolysezellen durchgeführt werden. Es sind somit geteilte und ungeteilte Zellen geeignet. Bevorzugt elektrolysiert man in einer ungeteilten Durchflußzelle. Als Anodenmaterialien werden z.B. Edelmetalle, wie Platin oder Metalloxide, wie $RuO_2$ eingesetzt. Bevorzugtes Anodenmaterial ist Graphit. Als Kathoden werden z.B. solche aus Eisen, Nickel,

Stahl oder Edelmetallen, wie Platin verwendet. Bevorzugt ist auch hier Graphit.

Für die Elektrooxidation werden als Elektrolyte Lösungen der 2-tert.-Butylphenylalkylether in Methanol bzw. Ethanol eingesetzt, die ein Leitsalz enthalten. Als Leitsalze kommen an sich übliche Leitsalze in Betracht, die unter den Elektrolysebedingungen weitgehend stabil sind, das sind z.B. Sulfate bzw. Alkylsulfate, wie Tetramethylammoniummethylsulfat, Sulfonate wie Kaliumbenzolsulfonat, Fluoride wie NaF und KF, Tetrafluoroborate wie $NaBF_4$, Hexafluorophosphate wie $KPF_6$ und Alkoholate, wie $NaOCH_3$. Weiterhin lassen sich auch Basen, wie KOH verwenden.

Der Elektrolyt hat beispielsweise folgende Zusammensetzung:

5 bis 49 Gew.% 2-tert.-Butylphenylalkylether
50 bis 95 Gew.% Methanol oder Ethanol
0,1 bis 5 Gew.% Leitsalz

Die Stromdichten liegen bei dem erfindungsgemäßen Verfahren zwischen 1 und 25 $A/dm^2$, vorzugsweise wird bei 2 bis 6 $A/dm^2$ gearbeitet. Man elektrolysiert zweckmäßigerweise bei Temperaturen bis 75 °C. Die Elektrolyse kann sowohl diskontinuierlich als auch kontinuierlich durchgeführt werden.

Die Aufarbeitung der Elektrolyseausträge erfolgt nach konventionellen Methoden, vorzugsweise wird destillativ aufgearbeitet. Überschüssiges Methanol (Ethanol), Leitsalz, unumgesetzter 2-tert.-Butylphenylalkylether sowie der als Zwischenstufe entstehende 2-tert.-Butylhydrochinondialkylether können zur Elektrolyse zurückgeführt werden.

Beispiel:

Apparatur: ungeteilte Zelle mit 11 bipolaren Elektroden
Anode: Graphit
Elektrolyt: 300 g 2-tert.-Butylanisol (10 %)
30 g KF (1 %) und 2670 g Methanol (89 %)
Kathode: Graphit
Stromdichte: 3,3 $A/dm^2$
Temperatur: 40 °C

Elektrolyse mit 8,2 F/mol 2-tert.-Butylanisol. Der Elektrolyt wird während der Elektrolyse mit 200 l/h durch die Zelle gepumpt. Nach Beendigung der Elektrolyse wird Methanol bei Normaldruck abdestilliert, das ausgefallene Leitsalz abfiltriert und das Filtrat im Vakuum reindestilliert. Man erhält 33,6 g (0,205 Mol) unumgesetztes 2-tert.-Butylanisol, 3,5 g (0,018 Mol) 2-tert.-Butylhydrochinondimethylether und 220,4 g (0,861 Mol) 2-tert.-Butyl-p-benzochinontetramethylketal [Sdp.: 105 °C/10 mbar, $^1H$-NMR ( $CDCl_3$): = 1,2 ( s,-$C(CH_3)_3$), 3,2, 3,3 ( s, -$OCH_3$), 5,9, 6,2 (m, arom.-H)].

Hieraus errechnet sich ein Umsatz (2-tert.-Butylanisol) von 88 %, eine Ausbeute an 2-tert.-Butyl-p-benzochinontetramethylketal von 47 % und eine Selektivität (2-tert.-Butyl-p-benzochinontetramethylketal) von 54 %.

## Ansprüche

1. 2-tert.-Butyl-p-benzochinontetraalkylketale der Formel

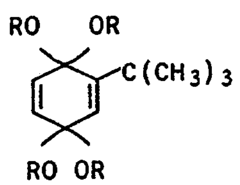

I,

in der R einen Methyl- oder Ethylrest bedeutet.

2. 2-tert.-Butyl-p-benzochinontetramethylketal

3. 2-tert.-Butyl-p-benzochinontetraethylketal

4. Verfahren zur Herstellung von 2-tert.-Butyl-p-benzochinontetraalkylketalen nach Anspruch 1, dadurch gekennzeichnet, daß man 2-tert.-Butylphenylalkylether der Formel

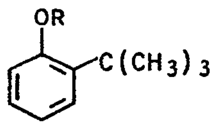

II,

in der R für einen Methyl- oder Ethylrest steht, in Gegenwart eines Alkohols ROH, in der R die genannte Bedeutung hat, elektrochemisch oxidiert.

5. Verfahren nach Anspruch 4, dadurch gekennzeichnet, daß man die elektrochemische Oxidation in ungeteilten Zellen an Graphitanoden durchführt.

6. Verfahren nach Anspruch 4, dadurch gekennzeichnet, daß man bei Temperaturen bis 75 °C und bei Stromdichten von 1-25 A/dm$^2$ arbeitet.

7. Verfahren nach Anspruch 4, dadurch gekennzeichnet, daß man für die elektrochemische Oxidation einen Elektrolyten mit der Zusammensetzung 5 bis 49 Gew.% 2-tert.-Butylphenylalkylether, 50 bis 95 Gew.% Alkohol ROH und 0,1 bis 5 Gew.% eines Leitsalzes verwendet.

Europäisches Patentamt

# EUROPÄISCHER RECHERCHENBERICHT

Nummer der Anmeldung

EP 90 12 2258

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF THE CHEMICAL SOCIETY, PERKIN TRANSACTIONS I, Nr. 7, 1978, Seiten 708-715; A. NILSSON et al.: "Anodic functionalisation in synthesis. Part 1. Methoxylation of methyl-substituted benzene and anisole derivatives, and the synthesis of aromatic aldehydes by anodic oxidation" * Schema 2, Seite 709 * | 1-7 | C 07 C 43/305<br>C 25 B 3/02 |
| A | FR-A-2 295 138 (HOECHST) * Ansprüche * | 1-7 | |
| A | EP-A-0 252 284 (BASF) * Ansprüche * | 1-7 | |
| | | | **RECHERCHIERTE SACHGEBIETE (Int. Cl.5)** |
| | | | C 07 C 43/00<br>C 07 C 41/00<br>C 25 B 3/00 |

Der vorliegende Recherchenbericht wurde für alle Patentansprüche erstellt

| Recherchenort | Abschlußdatum der Recherche | Prüfer |
|---|---|---|
| DEN HAAG | 14-02-1991 | ZERVAS B. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)